(19) Europäisches Patentamt
European Patent Office
Office européen des brevets

(11) Veröffentlichungsnummer: **0 325 962 B1**

# (12) EUROPÄISCHE PATENTSCHRIFT

(45) Veröffentlichungstag der Patentschrift: **07.12.94**

(51) Int. Cl.$^5$: **B01D 71/00**, B01D 71/56, C08G 69/32

(21) Anmeldenummer: **89100537.3**

(22) Anmeldetag: **13.01.89**

## (54) Makroporöse, asymmetrische, hydrophile Membran aus Polyaramid.

(30) Priorität: **25.01.88 DE 3802030**

(43) Veröffentlichungstag der Anmeldung:
**02.08.89 Patentblatt 89/31**

(45) Bekanntmachung des Hinweises auf die Patenterteilung:
**07.12.94 Patentblatt 94/49**

(84) Benannte Vertragsstaaten:
**AT BE CH DE ES FR GB GR IT LI LU NL SE**

(56) Entgegenhaltungen:
**EP-A- 0 027 920**
**EP-A- 0 199 090**
**US-A- 4 619 932**

(73) Patentinhaber: **HOECHST AKTIENGESELL-SCHAFT**

**D-65926 Frankfurt (DE)**

(72) Erfinder: **Haubs, Michael, Dr.**
**Auf dem Weiher 18**
**D-6550 Bad Kreuznach (DE)**
Erfinder: **Herold, Friedrich, Dr.**
**Thüringer Weg 57**
**D-6238 Hofheim (DE)**
Erfinder: **Krieg, Claus-Peter, Dr.**
**Im Mainfeld 42**
**D-6000 Frankfurt 71 (DE)**
Erfinder: **Skaletz, Detlef, Dr.**
**Südring 281**
**D-6500 Mainz-Bretzenheim (DE)**
Erfinder: **Wildhardt, Jürgen**
**Am Südhang 32**
**D-6274 Hünstetten 2 (DE)**

EP 0 325 962 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft makroporöse, asymmetrische, hydrophile Membranen enthaltend Polyaramid sowie ein Verfahren zu ihrer Herstellung.

Seit der Einführung asymmetrischer Membranen aus Celluloseacetat durch Loeb und Sourirajan, (S. Sourirajan, Reverse Osmosis, Logos Press, London 1970) und aus hydrophoben Polymerisaten (US-A-3,615,024) sind zahlreiche Membranen, insbesondere für Separationen von in Wasser gelösten nieder- und makromolekularen Bestandteilen entwickelt und vorgeschlagen worden, deren Struktur und Eignung in der Literatur angegeben (Desalination, 35 (1980), 5-20) und die auch in industrieller Praxis oder für medizinische Zwecke mit Erfolg erprobt wurden.

Viele von den beschriebenen Membranen haben zur Lösung spezifischer Aufgaben besonders vorteilhafte Eigenschaften. Infolge ihres chemischen Aufbaus und ihrer baulichen Struktur können die einzelnen Membranen jeweils nur für ganz spezielle Separationsprobleme optimal geeignet sein. Hieraus resultiert die grundlegende Forderung, für neue Aufgabenstellungen stets neue Membranen zu entwickeln.

In der EP-A-0 082 433 wird eine übersichtliche Darstellung über die Vor- und Nachteile bereits bekannter Membranen gegeben. So gibt es beispielsweise hydrophile, asymmetrische Membranen aus Celluloseacetat mit befriedigenden antiadsorptiven Eigenschaften, die aber bezüglich ihrer thermischen und chemischen Beständigkeit sehr zu wünschen lassen. Membranen aus Polysulfonen oder ähnlichen Polymeren besitzen zwar eine gute thermische und chemische Beständigkeit, allerdings besteht bei solchen Membranen wegen der hydrophoben Eigenschaften der eingesetzten Polymeren eine ausgeprägte Neigung, gelöste Stoffe zu adsorbieren, wodurch sich die Membran quasi verstopft. Die in der EP-A-0 082 433 beschriebenen Mischungen aus Polysulfon und Polyvinylpyrrolidon beseitigen zwar den durch die Hydrophobie des Polysulfons bedingten Nachteil, diese Mischungen sind jedoch gegenüber der Einwirkung von organischen Lösemitteln empfindlich.

Hydrophilie und gleichzeitig Resistenz gegenüber Lösemitteln findet man bei Membranen aus Celluloseregenerat; diese sind aber in sauren oder alkalischen Medien relativ leicht hydrolysierbar, und darüber hinaus werden sie von Mikroorganismen leicht angegriffen.

Der Erfindung liegt die Aufgabe zugrunde, makroporöse asymmetrische Membranen bereitzustellen, die ausgeprägte hydrophile Eigenschaften besitzen, d.h. befähigt sind, erhebliche Mengen Wasser, bezogen auf ihr Gesamtgewicht, aufzunehmen, die gegen verseifende sowie gegen oxidative Agenzien sowie gegenüber thermischer Einwirkung beständig sind, die organischen Lösemitteln besser widerstehen als Membranen aus hydrophobem Polymerisat, die gute Benetzbarkeit aufweisen und die auch gegenüber der Einwirkung von Mikroorganismen unempfindlich sind.

Gelöst wird diese Aufgabe durch eine Membran der eingangs genannten Gattung, deren Kennzeichenmerkmale darin zu sehen sind, daß sie ein Copolyamid enthält, das wenigstens die folgenden wiederkehrenden Struktureinheiten aufweist:

(A) -OC-Ar-CO-

(B) -NH-Ar′-NH-

(C) 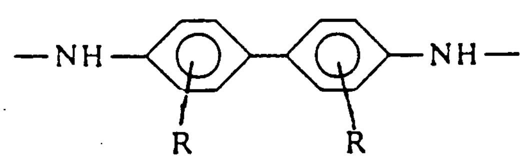

(D) 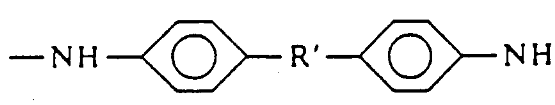

Dabei bedeuten

| | |
|---|---|
| Ar und Ar′ | zweiwertige aromatische Reste, bei denen die Valenzbindungen in para- oder vergleichbarer coaxialer oder paralleler Stellung stehen, |
| R | einen niederen Alkylrest oder einen niederen Alkoxyrest mit jeweils bis zu 4 C-Atomen oder einen Halogenrest und |
| R′ | einen unsubstituierten oder substituierten Methylenrest oder eine Gruppierung - O - Ar - O -, wobei Ar den gleichen Aufbau wie oben angegeben aufweist. |

Erfindungsgemäß sind also zur Bildung der in der Membran enthaltenen Copolyamide drei Diaminkomponenten notwendig, wobei die Kettenvalenzbindungen an den einzelnen Benzolringen jeweils nur in para-Position oder entsprechender coaxialer bzw. paralleler Stellung stehen sollen. Die Mengen der eingesetzten Diamine sind nicht beliebig wählbar, sondern um optimale Membraneigenschaften zu erreichen, sollen die Konzentrationen der Diamine (B) im Bereich von 5 bis 60 Mol-%, der Diamine (C) im Bereich von 5 bis 80 Mol-% und der Diamine (D) im Bereich von 5 bis 50 Mol-% liegen, bezogen auf 100 Mol-% eingesetzte Säurekomponente (A).

Die bevorzugten Bereiche der Konzentrationen der Diamine betragen für Diamin (B) 15 bis 50 Mol-%, für Diamin (C) 20 bis 75 Mol-% und für Diamin (D) 10 bis 40 Mol-%, ebenfalls bezogen auf 100 Mol-% eingesetzte Säurekomponente (A).

Zur Herstellung der erfindungsgemäß benötigten Copolyamide sind die folgenden Verbindungen geeignet:

Als Dicarbonsäurederivate der Formel

(A′) Cl - CO - Ar - CO - Cl

sind beispielsweise geeignet 4,4′-Diphenylsulfondicarbonsäuredichlorid, 4,4′-Diphenyletherdicarbonsäuredichlorid, 4,4′-Diphenyldicarbonsäuredichlorid, 2,6-Naphthalindicarbonsäuredichlorid, ganz besonders aber Terephthalsäuredichlorid.

Als aromatisches Diamin der Struktur

(B′) $H_2N$ - Ar′ - $NH_2$

eignet sich insbesondere p-Phenylendiamin.

Als Benzidinderivate (C′) der Formel

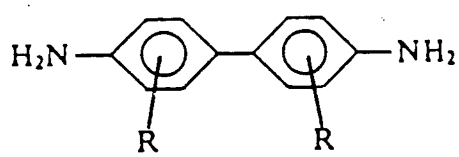

eignen sich besonders das 3,3′-Dimethoxy-, das 3,3′-Dichlor- und ganz besonders das 3,3′-Dimethylbenzidin.

Von der Diaminkomponente (D′) der Formel

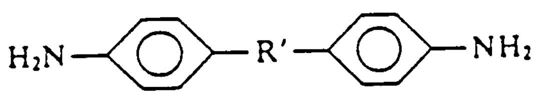

seien besonders das 4,4′-Diaminodiphenylmethan, das 2,2-bis-(4-Aminophenyl)-propan und ganz besonders das 1,4-bis-(4-Aminophenoxy)-benzol genannt.

Die Lösungskondensation der aromatischen Dicarbonsäuredichloride mit den Mischungen aus aromatischen Diaminen erfolgt in aprotischen, polaren Lösungsmitteln vom Amidtyp wie z.B. in N,N-Dimethylacetamid oder insbesondere in N-Methyl-2-pyrrolidon. Gegebenenfalls können diesen Lösungsmitteln in bekannter Weise zur Erhöhung der Lösefähigkeit bzw. zur Stabilisierung der Polyamidlösungen Halogenidsalze der ersten und zweiten Gruppe des periodischen Systems zugegeben werden. Bevorzugte Zusätze sind Calciumchlorid und/oder Lithiumchlorid.

Die Polykondensationstemperaturen liegen üblicherweise zwischen -20 °C und +120 °C, bevorzugt zwischen +10 °C und +100 °C. Besonders gute Ergebnisse werden bei Reaktionstemperaturen zwischen +10 °C und +80 °C erzielt. Die Polykondensationsreaktionen werden vorzugsweise so ausgeführt, daß nach Abschluß der Reaktion 2 bis 15, vorzugsweise 3,5 bis 10 Gew.-% an Polykondensat in der Lösung vorliegen. Wenn allerdings der Anteil der Komponente "D" sich 10 % nähert, muß die Polymerkonzentration in der Lösung deutlich herabgesetzt werden.

Die Polykondensation kann in üblicher Weise z. B. durch Zugabe von monofunktionellen Verbindungen wie z. B. Benzoylchlorid gestoppt werden.

Nach Beendigung der Polykondensation, d. h. wenn die Polymerlösung den zur Weiterverarbeitung erforderlichen Staudinger-Index erreicht hat, wird der entstandene und locker an das Amidlösungsmittel gebundene Chlorwasserstoff neutralisiert durch Zugabe basischer Substanzen. Geeignet sind dafür beispielsweise Lithiumhydroxyd, Calciumhydroxyd, insbesondere aber Calciumoxid.

Der Staudinger-Index ist ein Maß für die mittlere Kettenlänge der entstandenen Polymeren.

Der Staudinger-Index der membranbildenden Copolyamide soll zwischen 50 und 1 000 $cm^3/g$, bevorzugt zwischen 100 und 500 $cm^3/g$, besonders bevorzugt zwischen 150 und 350 $cm^3/g$, liegen. Er wurde bestimmt an Lösungen von jeweils 0,5 g Polymer in 100 ml 96%iger Schwefelsäure bei 25 °C.

Unter Staudinger-Index $[\eta]$ (Grenzviskosität, intrinsic viscosity) wird der Ausdruck

$$\lim_{C_2 \to 0} \frac{\eta_{sp}}{C_2} = [\eta]$$

verstanden, wobei

$\eta_{sp}$ = spezifische Viskosität =

$$\frac{\eta}{\eta_1} - 1$$

$C_2$ = Konzentration des gelösten Stoffes

$\eta$ = Viskosität der Lösung

$\eta_1$ = Viskosität des reinen Lösemittels

ist.

Die im vorstehenden beschriebenen Copolyamide sind als solche nicht Gegenstand der vorliegenden Erfindung, sondern diese sind bereits in der DE-A-36 05 394 im Zusammenhang mit Formkörpern beschrieben. Die Erfindung betrifft vielmehr eine semipermeable makroporöse Membran enthaltend die genannten Copolyamide als Hauptbestandteil.

Zur Herstellung der erfindungsgemäßen Membran aus den Copolyamiden wird die bereits beschriebene Lösung der Copolyamide filtriert, entgast, und dann wird in bekannter Weise nach dem Phaseninversionsverfahren (Robert E. Kesting, "Synthetic Polymeric Membranes", 2nd Ed., 1985, S. 237 ff.) eine asymmetrische makroporöse Membran hergestellt. Zu diesem Zweck wird die Polymerisatlösung auf eine möglichst plane Unterlage als flüssige Schicht ausgebreitet. Die plane Unterlage kann beispielsweise aus einer Glasplatte oder aus einer Metalltrommel bestehen.

Danach läßt man auf die flüssige Schicht Fällflüssigkeit einwirken, die mit dem Lösungsmittel der Lösung mischbar ist, in der aber die in der Polymerisatlösung gelösten Polymerisate als Membran ausgefällt werden. Als Fällflüssigkeit wird beispielsweise Wasser verwendet. Durch die Einwirkung der Fällflüssigkeit auf die flüssige Schicht aus Polymerenlösung werden die in dieser gelösten Copolyamide unter Ausbildung einer makroporösen Folie mit asymmetrischer Porenstruktur ausgefällt.

Bei der Verfahrensdurchführung läßt man die Fällflüssigkeit vorteilhaft so lange auf die durch diese ausgefällte Membran einwirken, bis aus dieser praktisch das gesamte Lösungsmittel durch Fällflüssigkeit ersetzt ist. Danach wird die gebildete Membran von Fällflüssigkeit befreit, beispielsweise dadurch, daß man die Membran direkt in einem Luftstrom trocknet oder aber zunächst mit einem Weichmacher wie Glycerin behandelt und danach trocknet.

Zur Herstellung von Membranen, die auf einer Trägerschicht angeordnet sind, welche für strömungsfähige Medien durchlässig ist, geht man wie vorstehend angegeben vor, verwendet jedoch als Unterlage zur Ausbildung der Membranschicht als Träger für diese ein Vlies, z. B. aus Kunststoff, oder ein Papier und beläßt nach Ausbildung der Membranschicht diese auf der Unterlage. Die Membran kann aber auch zunächst trägerfrei hergestellt und erst danach auf einen durchlässigen Träger aufgebracht werden.

In bekannter Weise sind aus der Lösung der Copolyamide auch Hohlfäden bzw. Kapillaren herstellbar, indem man die Polymerisatlösung nach dem Stande der Technik durch eine entsprechend ausgebildete formgebende Ringspalt-bzw. Hohlnadeldüse in Fällflüssigkeit einspinnt. Die Wandstärke derartiger Kapillaren oder Hohlfasern liegt üblicherweise im Bereich von 20 bis 80 µm.

Wird die Membran nach der Koagulation mit Glycerin getränkt, so kann sie vorzugsweise im Bereich von 5 bis 60 % Glycerin, bezogen auf ihr Gesamtgewicht, enthalten; die derart imprägnierte Membran wird getrocknet, z. B. bei einer Temperatur von 50 °C.

Die erfindungsgemäße Membran eignet sich ebenfalls als Trägermembran für permselektive Schichten, die direkt auf oder in der Membran erzeugt werden. So können beispielsweise "ultradünne" Schichten (≦ 1 µm) aus Polymeren mit funktionellen Gruppen (z. B. Silikone, Celluloseether, Fluorcopolymere) auf Wasser gespreitet werden, von dort auf die Membranoberfläche aufgebracht und z. B. durch Reaktion mit einem Diisocyanat kovalent fixiert werden, um somit höhere Permselektivitäten zu erzielen. Analog eignet sich die erfindungsgemäße Membran auch als Träger reaktiver Moleküle, beispielsweise um Enzyme oder Antikoagulantien wie Heparin nach dem Stande der Technik zu fixieren.

Die Dicke der erfindungsgemäßen Membran ohne Trägerschicht liegt im Bereich von 10 bis 300 µm, insbesondere von 20 bis 120 µm.

Die Erfindung soll nachfolgend anhand von Ausführungsbeispielen noch eingehender dargestellt werden, ohne jedoch auf die dort angegebenen Ausführungsformen beschränkt zu sein.

Beispiele 1 bis 4

Für die Herstellung der in den Beispielen untersuchten Membran wurde in N-Methylpyrrolidon als Lösemittel ein Copolyamid aus

(A′) 100 Mol-% Terephthalsäuredichlorid (TPC),
(B′) 25 Mol-% Paraphenylendiamin (PPD),
(C′) 50 Mol-% 3,3′-Dimethylbenzidin (DMB) und
(D′) 25 Mol-% 1,4-bis-(4-Aminophenoxy)-benzol (BAPOB)

bei einer Temperatur von 50 °C hergestellt. Lösungen dieses Copolyamids mit verschiedenen Staudinger-Indices und von verschiedener Konzentration (genauere Angaben siehe Tabelle 1) wurden dann mit einer Gießvorrichtung gemäß US-A-4 229 291 auf ein Polypropylen-Trägervlies (erhältlich bei Fa. Freudenberg: FO 2430(R) 100 g/m$^2$) aufgebracht und in Wasser bei 14 °C koaguliert. Die Membranen sind dann mit einer wäßrigen Lösung von 40 Gew.-% Glycerin getränkt und bei 50 °C getrocknet worden. Die trockenen trägerverstärkten Membranen besaßen eine Dicke von 280 µm.

Durch eine Wärmebehandlung der Membran ist überraschenderweise eine nachträgliche Veränderung der Membraneigenschaften zu erreichen. In Beispiel 4 wird gezeigt, wie durch Einlegen der Membran in heißes Wasser (80 °C) eine sehr wesentliche Erhöhung des Rückhaltevermögens für gelöste Substanzen ermöglicht wird.

Die Membraneigenschaften der so hergestellten Membranen sind in der nachfolgenden Tabelle 1 angegeben.

- Der Staudinger-Index wurde wie in der Beschreibung angegeben in 96 % $H_2SO_4$ bei 25 °C bestimmt.
- Die mechanische Permeabilität (Ultrafiltration) und das Rückhaltevermögen gegenüber gelösten Makromolekülen wurden bei Drucken von 3,0 bar bei 20 °C in einer gerührten zylindrischen Zelle bestimmt (700 U/min, 350 ml, Membranfläche 43 cm$^2$).

  Das Rückhaltevermögen ist definitionsgemäß

$$R = \frac{C_1 - C_2}{C_1} \cdot 100 \quad [\%]$$

  $C_1$ ist die Konzentration der wäßrigen Testlösung,
  $C_2$ ist die Konzentration im Permeat.

Als Testlösung wurde eine 2%ige wäßrige Polyvinylpyrrolidonlösung (PVP) eingesetzt, erhältlich unter dem Namen "Kollidon K30"(R) der Firma BASF, die Molmasse des Polyvinylpyrrolidons betrug 49 000 Dalton.

Die Konzentrationsmessungen erfolgten in einem digitalen Dichtemeßgerät "DMA 60 + 601"(R) der Firma Heraeus.

Tabelle 1

| Beispiel | TPC (Mol-%) | PPD (Mol-%) | BAPOB (Mol-%) | DMB (Mol-%) | $CaCl_2$ GT[1)] | Staudinger-Index ($cm^3/g$) | Polymerkonzentration (%) | mechanische Permeabilität ($l/m^2h$) | Rückhaltevermögen (%) | Testsubstanz |
|---|---|---|---|---|---|---|---|---|---|---|
| 1 | 100 | 25 | 25 | 50 | 3,0 | 188 | 7,0 | 97,5 | 92 | PVP K30 |
| 2 | 100 | 25 | 25 | 50 | 3,0 | 328 | 6,0 | 306 | 75 | PVP K30 |
| 3 | 100 | 25 | 25 | 50 | 3,0 | 188 | 4,67 | 216 | 85 | PVP K30 |
| 4[2)] | 100 | 25 | 25 | 50 | 3,0 | 188 | 7,0 | 63 | 99,5 | PVP K30 |
| | | | | | | | | | 99,0 | PEG[3)] 10 000 |

1) Gewichtsteile, bezogen auf 100 Gewichtsteile Polymerlösung
2) thermische Nachbehandlung der Membranen aus Beispiel 3: 48 h 80 °C in Wasser
3) Polyethylenglykol, Molmasse 10 000

Beispiele 5 bis 9

Um die Lösemittelbeständigkeit der erfindungsgemäßen Membranen zu testen, wurden die Membranen der Beispiele 1 bis 4 l h in Aceton gelegt, um die in den Poren der Membranen enthaltene Flüssigkeit

gegen Aceton auszutauschen. Danach wurden die Membranen über einen Zeitraum von 12 h den in Tabelle 2 angegebenen Lösemitteln bei einer Temperatur von 25 °C ausgesetzt. Im Anschluß daran wurden die Membranen auf Wasser umkonditioniert, und es wurde wie unter Beispiel 1 angegeben die mechanische Permeabilität und das Rückhaltevermögen an den mit den organischen Lösemitteln behandelten Membranen gemessen. Die Ergebnisse sind in Tabelle 2 dargestellt und zeigen, daß die Abweichungen von den in Tabelle 1 angegebenen Werten innerhalb der Toleranzgrenzen des Meßverfahrens liegen.

Tabelle 2

| Beispiel | Membran aus Beispiel | Lösemittel | mech.Permeabilität ($l/m^2h$) | Rückhaltevermögen (%) | Testsubstanz |
|---|---|---|---|---|---|
| 5 | 1 | THF[1)] | 92,3 | 93 | PVP K30 |
| 6 | 1 | $CH_2Cl_2$ | 90,5 | 92,5 | PVP K30 |
| 7 | 2 | $CHCl_3$ | 301 | 76 | PVP K30 |
| 8 | 2 | Toluol | 288 | 75,5 | PVP K30 |
| 9 | 3 | EE[2)] | 199 | 86,5 | PVP K30 |

[1)] Tetrahydrofuran

[2)] Essigsäureethylester

**Patentansprüche**

**1.** Makroporöse, asymmetrische, hydrophile Membran enthaltend Polyaramid, dadurch gekennzeichnet, daß sie ein Copolyamid enthält, das wenigstens die folgenden wiederkehrenden Struktureinheiten aufweist:

(A) -OC-Ar-CO-

(B) -NH-Ar′-NH-

(C) 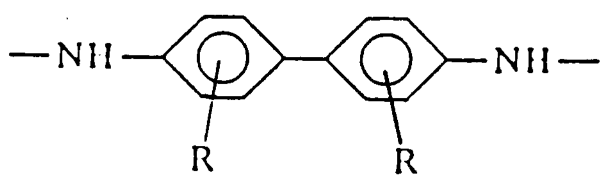

(D) 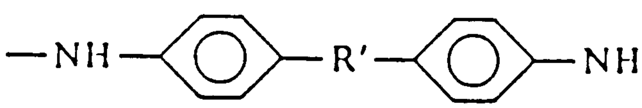

wobei

| | |
|---|---|
| Ar und Ar′ | zweiwertige aromatische Reste, bei denen die Valenzbindungen in para- oder vergleichbarer coaxialer oder paralleler Stellung stehen, |
| R | einen niederen Alkylrest oder einen niederen Alkoxyrest mit jeweils bis zu 4 C-Atomen oder einen Halogenrest und |
| R′ | einen unsubstituierten oder substituierten Methylenrest oder eine Gruppierung - O - Ar - O -, wobei Ar den gleichen Aufbau wie oben angegeben aufweist, |

bedeuten.

**2.** Membran nach Anspruch 1, dadurch gekennzeichnet, daß die Konzentration der Diaminkomponente (B) im Bereich von 5 bis 60 Mol-%, der Diaminkomponente (C) im Bereich von 5 bis 80 Mol-% und der Diaminkomponente (D) im Bereich von 5 bis 50 Mol-% liegt, bezogen auf 100 Mol-% eingesetzte Säurekomponente (A).

3. Membran nach Anspruch 1, dadurch gekennzeichnet, daß die bevorzugte Konzentration der Diaminkomponente (B) 15 bis 50 Mol-%, der Diaminkomponente (C) 20 bis 75 Mol-% und der Diaminkomponente (D) 10 bis 40 Mol-% beträgt, bezogen auf 100 Mol-% eingesetzte Säurekomponente (A).

4. Membran nach einem der Ansprüche 1 bis 3, dadurch gekennzeichnet, daß als Carbonsäurederivatkomponente (A) Verbindungen geeignet sind wie beispielsweise 4,4′-Diphenylsulfondicarbonsäuredichlorid, 4,4′-Diphenyletherdicarbonsäuredichlorid, 4,4′-Diphenyldicarbonsäuredichlorid, 2,6-Naphthalindicarbonsäuredichlorid, ganz besonders aber Terephthalsäuredichlorid.

5. Membran nach einem der Ansprüche 1 bis 4, dadurch gekennzeichnet, daß als Diaminkomponente (B) insbesondere p-Phenylendiamin geeignet ist.

6. Membran nach einem der Ansprüche 1 bis 5, dadurch gekennzeichnet, daß als Diaminkomponente (C) Verbindungen geeignet sind wie beispielsweise 3,3′-Dimethoxy-, 3,3′-Dichlor- und ganz besonders 3,3′-Dimethylbenzidin.

7. Membran nach einem der Ansprüche 1 bis 6, dadurch gekennzeichent, daß als Diaminkomponente (D) Verbindungen geeignet sind wie beispielsweise 4,4′-Diaminodiphenylmethan, 2,2-Bis-(4-Aminophenyl)-propan und ganz besonders 1,4-Bis-(4-Aminophenoxy)-benzol.

8. Membran nach einem der Ansprüche 1 bis 7, dadurch gekennzeichnet, daß das Copolyamid einen Staudinger-Index im Bereich von 50 bis 1 000 $cm^3/g$, bevorzugt von 100 bis 500 $cm^3/g$, besonders bevorzugt von 150 bis 350 $cm^3/g$, aufweist, gemessen in 96%iger Schwefelsäure bei 20 °C.

9. Membran nach einem der Ansprüche 1 bis 8, dadurch gekennzeichnet, daß sie eine Flachmembran ist mit einer Dicke im Bereich von 10 bis 300 µm, vorzugsweise im Bereich von 20 bis 150 µm.

10. Membran nach Anspruch 9, dadurch gekennzeichnet, daß sie auf einer für fließfähige Medien durchlässigen Trägerschicht aus Kunststoffvlies oder aus Papier angeordnet ist.

11. Membran nach einem der Ansprüche 1 bis 8, dadurch gekennzeichnet, daß sie eine Hohlfasermembran ist.

12. Membran nach einem der Ansprüche 1 bis 11, dadurch gekennzeichnet, daß sie eine Menge von 5 bis 60 Gew-% Glycerin enthält, bezogen auf das Gesamtgewicht der Membran.

13. Verfahren zur Herstellung einer Membran nach einem der Ansprüche 1 bis 12, bei dem die Polymerisatlösung auf eine plane Unterlage als flüssige Schicht ausgebreitet und danach auf die flüssige Schicht Fällflüssigkeit aufgebracht wird, die mit dem Lösungsmittel der Lösung mischbar ist, in der aber die in der Polymerisatlösung gelösten Polymerisate als Membran ausgefällt werden, wobei als Lösemittel für die Copolyamide aprotische, polare Lösemittel vom Amidtyp wie N,N-Dimethylacetamid oder insbesondere N-Methyl-2-pyrrolidon verwendet werden.

14. Verfahren nach Anspruch 13, dadurch gekennzeichnet, daß als Fällflüssigkeit Wasser verwendet wird.

15. Verfahren nach Anspruch 13 oder 14, dadurch gekennzeichnet, daß man die Fällflüssigkeit so lange auf die durch diese ausgefällte Membran einwirken läßt, bis aus dieser praktisch das gesamte Lösungsmittel durch Fällflüssigkeit ersetzt ist.

16. Verfahren nach einem der Ansprüche 13 bis 15, dadurch gekennzeichnet, daß die Membran von Fällflüssigkeit befreit wird, indem sie in einem Luftstrom getrocknet wird.

17. Verfahren nach einem der Ansprüche 13 bis 16, dadurch gekennzeichnet, daß die Membran vor dem Trocknen mit einem Weichmacher wie Glycerin behandelt und danach getrocknet wird.

18. Verfahren nach Anspruch 16 oder 17, dadurch gekennzeichnet, daß die Membran bei einer Temperatur von 50 °C getrocknet wird.

**19.** Verfahren nach Anspruch 15, dadurch gekennzeichnet, daß die Membran, einer Wärmebehandlung in einer Flüssigkeit unterzogen wird.

**20.** Verfahren nach Anspruch 19, dadurch gekennzeichnet, daß die Wärmebehandlung in einer inerten Flüssigkeit durchgeführt wird.

**21.** Verfahren nach Anspruch 15, dadurch gekennzeichnet, daß die Membran, einer Wärmebehandlung mit Wasserdampf unterzogen wird.

**22.** Verfahren nach Anspruch 17, dadurch gekennzeichnet, daß die Membran einer Wärmebehandlung in warmer Luft mit einer rel. Luftfeuchtigkeit von 20 bis 100 % unterzogen wird.

**23.** Verfahren nach einem der Ansprüche 19 bis 22, dadurch gekennzeichnet, daß die Wärmebehandlung bei einer Temperatur im Bereich von 60 bis 220 °C über einen Zeitraum von 0,1 bis 96 h erfolgt.

## Claims

**1.** A macroporous, asymmetric, hydrophilic membrane containing polyaramide, characterized in that it contains a copolyamide having at least the following recurring structural units:

(A) -OC-Ar-CO-

(B) -NH-Ar'-NH-

(C)

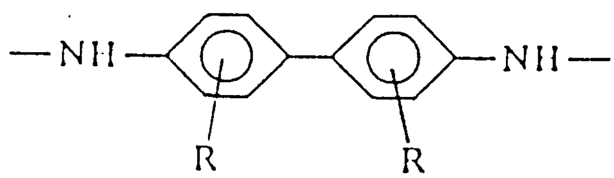

(D)

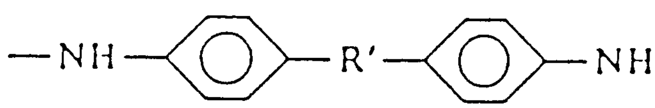

where

| | |
|---|---|
| Ar and Ar' | denote divalent aromatic radicals in which the valence bonds are in the para or comparable coaxial or parallel position, |
| R | denotes a lower alkyl radical or a lower alkoxy radical in each case having up to 4 carbon atoms, or a halogen radical, and |
| R' | denotes an unsubstituted or substituted methylene radical or an - O - Ar - O - group where Ar has the structure as given above. |

**2.** A membrane as claimed in claim 1, wherein the concentration of the diamine components (B) is in the range of 5 to 60 mol-%, the concentration of the diamine component (C) is in the range of 5 to 80 mol-% and the concentration of the diamine component (D) is in the range of 5 to 50 mol-%, based on 100 mol-% of the acid component (A) employed.

**3.** A membrane as claimed in claim 1, wherein the preferred concentration of the diamine component (B) is 15 to 50 mol-%, the preferred concentration of the diamine component (C) is 20 to 75 mol-% and the preferred concentration of the diamine component (D) is 10 to 40 mol-%, based on 100 mol-% of the acid component (A) employed.

**4.** A membrane as claimed in any one of claims 1 to 3, wherein suitable carboxylic acid derivative components (A) are compounds such as, for example, 4,4'-diphenyl sulfone dicarbonyl dichloride, 4,4'-diphenyl ether dicarbonyl dichloride, 4,4'-diphenyldicarbonyl dichloride and 2,6-naphthalenedicarbonyl dichloride, but very particularly terephthaloyl dichloride.

5. A membrane as claimed in any one of claims 1 to 4, wherein a suitable diamine component (B) is, in particular, p-phenylenediamine.

6. A membrane as claimed in any one of claims 1 to 5, wherein suitable diamine components (C) are compounds such as, for example, 3,3'-dimethoxybenzidine,3,3'-dichlorobenzidine and very particularly 3,3'-dimethylbenzidine.

7. A membrane as claimed in any one of claims 1 to 6, wherein suitable diamine components (D) are compounds such as, for example, 4,4'-diaminodiphenylmethane, 2,2-bis-(4-aminophenyl)propane and very particularly 1,4-bis-(4-aminophenoxy)benzene.

8. A membrane as claimed in any one of claims 1 to 7, wherein the copolyamide has a Staudinger index in the range from 50 to 1,000 $cm^3/g$, preferably 100 to 500 $cm^3/g$, particularly preferably 150 to 350 $cm^3/g$, measured in 96 % strength of sulfuric acid at 20 °C.

9. A membrane as claimed in any one of claims 1 to 8, being a flat membrane having a thickness in the range from 10 to 300 µm, preferably in the range from 20 to 150 µm.

10. A membrane as claimed in claim 9, which is arranged on a support layer which is permeable to flowable media and is made from a plastic nonwoven or paper.

11. A membrane as claimed in any one of claims 1 to 8, being a hollow fiber membrane.

12. A membrane as claimed in any one of claims 1 to 11, containing 5 to 60 % by weight of glycerol, based on the total weight of the membrane.

13. A process for the production of a membrane as claimed in any one of claims 1 to 12, in which the polymer solution is spread as a liquid layer on a planar substrate, and precipitation liquid which is miscible with the solvent of the solution, but in which the polymers dissolved in the polymer solution are precipitated as a membrane is then applied to the liquid layer, which comprises using aprotic, polar solvents of the amide type, such as N,N-dimethylacetamide or, in particular, N-methyl-2-pyrrolidone as solvent for the copolyamides.

14. The process as claimed in claim 13, wherein the precipitation liquid used is water.

15. The process as claimed in claim 13 or 14, wherein the precipitation liquid is allowed to act on the membrane precipitated thereby until virtually all the solvent has been replaced therein by precipitation liquid.

16. The process as claimed in any one of claims 13 to 15, wherein the membrane is freed from precipitation liquid by being dried in a stream of air.

17. The process as claimed in any one of claims 13 to 16, wherein the membrane is treated, before drying, with a plasticizer, such as glycerol, and then dried.

18. The process as claimed in claim 16 or 17, wherein the membrane is dried at a temperature of 50 °C.

19. The process as claimed in claim 15, which comprises subjecting the membrane to heat treatment in a liquid.

20. The process as claimed in claim 19, wherein the heat treatment is carried out in an inert liquid.

21. The process as claimed in claim 15, which comprises subjecting the membrane to heat treatment with steam.

22. The process as claimed in claim 17, which comprises subjecting the membrane to heat treatment in warm air of relative atmospheric humidity of 20 to 100%.

23. The process as claimed in any one of claims 19 to 22, wherein the heat treatment is carried out at a temperature in the range from 60 to 220 ° C over a period of 0.1 to 96 hours.

**Revendications**

1. Membrane macroporeuse asymétrique hydrophile à base de polyaramide, caractérisée en ce qu'elle contient un copolyamide qui présente au moins les motifs structuraux récurrents suivants:

(A) -OC-Ar-CO-

(B) -NH-Ar'-NH-

(C) 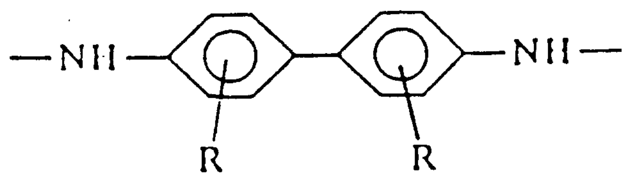

(D) 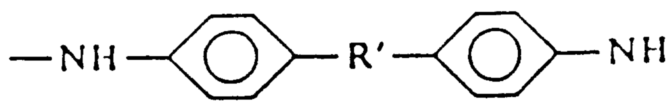

dans lesquels
Ar et Ar' représentent des restes aromatiques divalents, dont les liaisons de valence sont en position para ou en position coaxiale ou parallèle comparable,
R représente un radical alkyle inférieur ou un radical alcoxy inférieur comportant chacun jusqu'à 4 atomes de carbone, ou bien un atome d'halogène, et
R' représente un radical méthylène non substitué ou substitué ou un groupement -O-Ar-O-, le radical Ar présentant la même configuration que celle indiquée ci-dessus.

2. Membrane selon la revendication 1, caractérisée en ce que la concentration du composant diamine (B) est comprise entre 5 et 60 % en moles, la concentration du composant diamine (C) est comprise entre 5 et 80 % en moles et la concentration du composant diamine (D) est comprise entre 5 et 50 % en moles, par rapport à 100 % en moles de composant acide (A) introduit.

3. Membrane selon la revendication 1, caractérisée en ce que la concentration préférée du composant diamine (B) est comprise entre 15 et 50 % en moles, la concentration préférée du composant diamine (C) est comprise entre 20 et 75 % en moles et la concentration du composant diamine (D) est comprise entre 10 et 40 % en moles, par rapport à 100 % en moles de composant acide (A) introduit.

4. Membrane selon une quelconque des revendications 1 à 3, caractérisée en ce qu'on utilise comme composant dérivé d'acide carboxylique (A) approprié des composés tels que p.ex. le dichlorure d'acide 4,4'-diphénylsulfonedicarboxylique, le dichlorure d'acide 4,4'-diphénylétherdicarboxylique, le dichlorure d'acide 4,4'-diphényldicarboxylique, le dichlorure d'acide 2,6-naphtalènedicarboxylique, mais tout particulièrement le dichlorure d'acide téréphtalique.

5. Membrane selon une quelconque des revendications 1 à 4, caractérisée en ce qu'on utilise comme composant diamine (B) approprié en particulier la p-phénylènediamine.

6. Membrane selon une quelconque des revendications 1 à 5, caractérisée en ce qu'on utilise comme composant diamine (C) approprié des composés tels que p.ex. la 3,3'-diméthoxybenzidine, la 3,3'-dichlorobenzidine et, tout particulièrement, la 3,3'-diméthylbenzidine.

7. Membrane selon une quelconque des revendications 1 à 6, caractérisée en ce qu'on utilise comme composant diamine (D) approprié des composés tels que p.ex. le 4,4'-diaminodiphénylméthane, le 2,2-bis(4-aminophényl)propane et surtout le 1,4-bis(4-aminophénoxy)benzène.

**8.** Membrane selon une quelconque des revendications 1 à 7, caractérisée en ce que le copolyamide présente un indice de Staudinger compris entre 50 et 1000 $cm^3/g$, de préférence entre 100 et 500 $cm^3/g$, tout particulièrement entre 150 et 350 $cm^3/g$, déterminé dans de l'acide sulfurique 96 % à 20 °C.

**9.** Membrane selon une quelconque des revendications 1 à 8, caractérisée en ce qu'elle est une membrane plane dont l'épaisseur est comprise entre 10 et 300 µm, en particulier entre 20 et 150 µm.

**10.** Membrane selon la revendication 9, caractérisée en ce qu'elle est disposée sur une couche support perméable aux milieux fluides et formée de tissu non tissé synthétique ou de papier.

**11.** Membrane selon une quelconque des revendications 1 à 8, caractérisée en ce qu'elle est une membrane à fibres creuses.

**12.** Membrane selon une quelconque des revendications 1 à 11, caractérisée en ce qu'elle contient de 5 à 60 % en poids de glycérol par rapport au poids total de la membrane.

**13.** Procédé de fabrication d'une membrane selon l'une quelconque des revendications 1 à 12, dans lequel on étale la solution de polymère, à l'état de couche liquide, sur un substrat plan et on dépose ensuite, sur la couche liquide, du liquide de précipitation, qui est miscible avec le solvant de la solution mais dans lequel les polymères dissous dans la solution de polymère sont précipités sous forme de membrane, les solvants utilisés pour les copolyamides étant des solvants polaires aprotiques de type amide, tels que p.er. le N,N-diméthylacétamide ou surtout la N-méthylpyrrolidone.

**14.** Procédé selon la revendication 13, caractérisé en ce qu'on utilise l'eau comme liquide de précipitation.

**15.** Procédé selon la revendication 13 ou 14, caractérisé en ce qu'on laisse agir le liquide de précipitation sur la membrane précipitée par lui assez longtemps pour que pratiquement tout le solvant de celle-ci soit remplacé par du liquide de précipitation.

**16.** Procédé selon une quelconque des revendications 13 à 15, caractérisé en ce qu'on chasse le liquide de précipitation de la membrane en la séchant dans un courant d'air.

**17.** Procédé selon une quelconque des revendications 13 à 16, caractérisé en ce que la membrane est traitée, avant séchage, avec un plastifiant, tel que le glycérol, puis séchée.

**18.** Procédé selon la revendication 16 ou 17, caractérisé en ce que la membrane est séchée à une température de 50 °C.

**19.** Procédé selon la revendication 15, caractérisé en ce que la membrane est soumise à un traitement thermique dans un liquide.

**20.** Procédé selon la revendication 19, caractérisé en ce que le traitement thermique dans un liquide est effectué dans un liquide inerte.

**21.** Procédé selon la revendication 15, caractérisé en ce que la membrane est soumise à un traitement thermique avec de la vapeur d'eau.

**22.** Procédé selon la revendication 17, caractérisé en ce que la membrane est soumise à un traitement thermique à l'air chaud, avec une humidité relative de l'air de 20 à 100 %.

**23.** Procédé selon une quelconque des revendications 19 à 22, caractérisé en ce que le traitement thermique est réalisé à une température comprise entre 60 et 220 °C pendant une durée de 0,1 à 96 h.